# NEUE EUROPÄISCHE PATENTSCHRIFT

(11) **EP 0 589 348 B2**
(45) Veröffentlichungstag und Bekanntmachung des Hinweises auf die Entscheidung über den Einspruch: **30.05.2012**
(45) Hinweis auf die Patenterteilung: 19.01.2005
(21) Anmeldenummer: 93114884.5
(22) Anmeldetag: 16.09.1993
(51) Int. Cl.: G01N 33/543, G01N 33/569

(54) **Verfahren zur immunchemischen Quantifizierung von inaktivierten, immunreaktiven Antigenen**
Method for immunological quantification of inactivated antigenes
Procédé pour quantification immunologique des antigènes inactivés

(30) Priorität: 25.09.1992 DE 4232073
(43) Veröffentlichungstag der Anmeldung: 30.03.1994
(73) Patentinhaber: Novartis Vaccines and Diagnostics GmbH, 35041 Marburg (DE)
(72) Erfinder: Gröner, Albrecht, D-64342 Seeheim (DE); Diderrich, Gaston, D-35075 Gladenbach/Sinkershausen (DE)
(74) Vertreter: Hallybone, Huw George

(56) Entgegenhaltungen:
- EP-A- 0 786 472
- WO-A-88/08136
- WO-A-90/04182
- WO-A-91/00872
- DD-A- 275 120
- DD-A- 277 336
- US-A- 4 959 211
- DATABASE WPI Week 8628, Derwent Publications Ltd., London, GB; AN 86-178341 & JP-A-61 110 055 (DIAMALT ET AL.) 28. Mai 1986
- DATABASE WPI Week 8620, Derwent Publications Ltd., London, GB; AN 86-128255 & JP-A-61 066 163 (KAGAKU OYOBI KESSEI) 4. April 1986
- DATABASE WPI Week 198926, Derwent Publications Ltd., London, GB; Class B04, AN 1989-187891 'Prep. of antigen component in bovine leukaemia vaccine' & JP 1 125 329 A (JAPAN HOLSTEIN BREEDING; MITSUI PHARM INC; MITSUI TOATSU CHEM INC) 17 Mai 1989
- Arch. Virol., vol. 108, 1989, p. 169-182
- BIOLOGICALS Bd. 18, 1990, Seiten 321 - 330
- IMMUNOLOGY AND CELL BIOLOGY Bd. 70, 1992, Seiten 181 - 191
- ACTA VIROLOGY Bd. 22, 1978, Seiten 371 - 382
- BROWN L.E. ET AL: 'Antigenic determinants of influenza virus hemagglutinin. V. Antigenicity of the HA2 chain' J. IMMUNOLOGY Bd. 125, 1980, Seiten 1583 - 1588
- BIOLOGICALS Bd. 18, 1990, Seiten 271 - 280
- VOPR. VIRUSOL. Bd. 30, 1985, Seiten 672 - 675

## Beschreibung

Die Erfindung betrifft ein Verfahren zur immunchemischen Quantifizierung von inaktivierten, immunreaktiven Antigenen, die zur Hervorrufung einer Immunantwort in Säugetieren und Menschen verwendet werden.

Impfstoffe gegen Erkrankungen, die durch Viren oder Mikroorganismen verursacht werden, enthalten eine bestimmte Menge Antigen, dessen Applikation zu einer Immunantwort im geimpften Tier oder Mensch führt. Da diese Immunantwort dosisabhängig ist, ist eine bestimmte Menge Antigen in einem Impfstoff Voraussetzung für dessen Wirksamkeit. Der Nachweis des Antigen-Gehaltes im Impfstoff erfolgt bisher üblicherweise in in vivo-Schutzversuchen und in Zukunft vermehrt mit in vitro-Methoden; der Antigen-Gehalt wird während der Produktion vorzugsweise durch eine Proteinbestimmung, Keimzahl-/Partikelbestimmung oder Immunpräzipitationsreaktion bestimmt, um den Impfstoff zu mischen/einzustellen.

Mikroorganismen im Sinne dieser Erfindung sind pathogene Mikroorganismen und Viren gegen deren Pathogenität in Säugetieren und Menschen durch Impfung mit inaktivierten Mikroorganismus- bzw. Virusbestandteilen oder ganzen Mikroorganismen oder Viren, teilweise oder ganz Immunität erzielt werden kann. Wirksam ist dabei der Impfstoff durch seinen Gehalt an bestimmten antigenen Strukturen.

Der Nachweis dieser Antigene in einer Vaccine, insbesondere wenn sie durch geeignete Inaktivierungsmittel behandelt worden war, und die quantitative Bestimmung dieser Antigene mit immunologischen Methoden, gibt daher einen Hinweis auf die Wirksamkeit der Vaccine.

So muß z. B. aufgrund gesetzlicher und ähnlicher Vorschriften, z. B. CPMP-Richtlinien, der Gehalt an Hämagglutinin einer Influenza-Vaccine, die in der Regel aus drei verschiedenen Influenzavirus-Stämmen besteht, im Endbehälter mit einem Immunodiffusionstest oder einer anderen geeigneten Methode bestimmt werden (CPMP-Richtlinie). Der Immunodiffusionstest wird als "Single-Radial-Immunodiffusion-Technique" (SRD) generell angewandt (G.C. SCHILD et al. (1975), "A single-radial-immunodiffusion technique for the assay of influenza haemagglutinin. Proposals for an assay method for the haemagglutinin content of influenza vaccines", Bulletin of the World Health Organization 53, 223-231.).

Es hat sich nun gezeigt, daß einige Influenza-Vaccinen sich offensichtlich aufgrund der physikalischen Form des Hämagglutinins - große Molekülkomplexe, die während der Spaltung der Viren durch Lipidlösungsmittel und nachfolgende Inaktivierung mit Formaldehyd entstehen - im SRD nicht überprüfen lassen (GANDHI, S.S. (1978), "The effect of formaldehyde treatment of influenza virus on the assay of its haemagglutinin antigen content by the single-radial-immunodiffusion (SRD) technique", J. Biological Standardization 6, 121-126). Eine Erklärung kann sein, daß die großen Molekülkomplexe in der Agarose weniger weit diffundieren als kleinere Moleküle und so einen geringeren Antigen-Gehalt vortäuschen.

Aber auch Antigene anderer Impfstoffe gegen durch Viren oder Mikroorganismen verursachte Erkrankungen lassen sich häufig mit den bisher bekannten Methoden nicht oder nicht exakt quantifizieren. Der Erfindung liegt also die Aufgabe zugrunde, ein Quantifizierungsverfahren zu finden, das unabhängig von dem zur Inaktivierung der Vakzine verwendeten Verfahren sicher und zuverlässig vergleichbare Meßergebnisse mit Primärstandards liefert, wie z. B. den NIBSC-Reagenzien des National Institut for Biological Standards and Control (im Auftrag der WHO).

Überraschenderweise zeigt sich nun, daß ein Nachweisverfahren, bei dem das oder die nachzuweisenden Antigene durch Adsorption an eine feste Phase gebunden werden und der Anteil des gebundenen Antigens durch einen oder mehrere, direkt oder indirekt markierte Antikörper bestimmt wird, die gestellte Aufgabe erfüllt.

In J. Immunol. (1980) 125 1583-1588 wird ein Verfahren zur Identifikation von Antigendeterminanten von Influenzavirus-HA beschrieben. Hiernach wird HA mit Bromcyan gespalten und die Fähigkeit der Fragmente, mit gegen gereinigtes influenzavirus erzeugten Antikörpern eine Wechselwirkung einzugehen, wird beurteilt. Die Spaltung von HA mit Bromcyan führt nicht zur Bildung von Hämagglutininmolekülkomplexen.

In Acta. Virology (1978) 22 371-382 wird die Fähigkeit von gegen intaktes Influenzavirus oder gereinigtes HA erzeugten Kaninchenseren, eine Wechselwirkung mit gereinigtem HA1 und HA2 einzugehen, beurteilt. Das gereinigte HA1 und HA2 wird mittels Gelfiltration von gereinigtem HA in 6M Guanidin hergestellt. Dieses Dokument beschreibt, daß die Seren sowohl mit HA1 als auch mit HA2 eine Wechselwirkung eingehen. HA1 und HA2 sind eindeutig HA-Untereinheiten und nicht HA-Molekülkomplexe.

In Immunology and Cell Biology (1992) 70 181-191 wird die Wirksamkeit von Western-Blots für den Nachweis von Antikörperreaktionen bei der Maus und beim Menschen gegen die Influenzavirionproteine HA1, HA2 und ungespaltenes HA beurteilt. Die Proteine wurden aus gereinigtem Virus durch Vermischen mit SDS für die Elektrophorese unter reduzierenden und nichtreduzierenden Bedingungen und 2minütigem Kochen hergestellt. Die Inaktivierung mit SDS führt nicht zu Hämagglutininmolekülkomplexen.

In Vopr. Virusol. (1985) 30 672-675 wird ein Testsystem für den Nachweis und die quantitative Bestimmung von Influenzavirushämagglutinin (HA) in biologischen Proben mittels eines Festphasen-Enzymimmunassays beschrieben. Das HA-haltige Antigen wird immunchemisch an eine Polystyrolmatrix gebunden.

In Biological (1990) 18 321-330 wird ein Verfahren zur Bestimmung der Antigenität eines Tollwutimpfstoffs durch quantitative Bestimmung des hämagglutininartigen Glycoproteins, das dieser enthält, beschrieben. Das Virus wird mit BPL inaktiviert, einem Inaktivierungsverfahren, das nicht zur Bildung von molekularen Hämagglutininkomplexen führt.

Arch. Virol. (1989) 108 169-182 beschreibt einen immunologischen Nachweis von mittels Formaldehyd vernetzten Influenza-Hämagglutininen unter Verwendung eines Western-Blots von PAGE mit polyklonalen HA1- und HA2-spezifischen Antikörpern.

Gegenstand dieser Erfindung ist daher ein Verfahren zur immunchemischen Quantifizierung von inaktivierten immunreaktiven Hämagglutinin-Molekülkomplexen von Influenzaviren, das folgende Schritte umfaßt:
a) eine Probe, die einen oder mehrere zu bestimmende Hämagglutinin-Molekülkomplexe enthält, die während der Spaltung der Viren durch Lipidlösungsmittel und nachfolgender Inaktivierung mit Formaldehyd entstanden sind, wird mit einer proteinbindenden festen Phase inkubiert, wobei Hämagglutinin-Molekülkomplex an diese feste Phase physikalisch adsorbiert und nicht durch immunchemische Reaktion gebunden wird,
b) die flüssige und die feste Phase werden getrennt,
c) der adsorbierte Hämagglutinin-Molekülkomplex wird mit einem oder mehreren spezifischen Antikörpern, die direkt oder indirekt eine Markierung tragen, inkubiert,
d) die Menge der an den Hämagglutinin-Molekülkomplex gebundenen Markierung wird bestimmt, und
e) aus der Menge der gebundenen Markierung wird durch Vergleich mit einem oder mehreren Standardwerten die Menge des immunreaktiven Hämagglotinin-Molekülkomplexes bestimmt.

Bevorzugt ist dabei ein solches Verfahren, wobei Antigene eines Influenzavirus mittels Reaktion mit einem polyklonalen Antikörper oder einem monoklonalen, oder einer Mischung von monoklonalen Antikörpern, die die gleiche Markierung tragen, nachgewiesen werden.

Bevorzugt ist ferner ein solches Verfahren, wobei Antigene eines Influenzavirus mittels einer Reaktion mit spezifischen polyklonalen oder monoklonalen Antikörpern aus einer Mischung verschiedener Mikroorganismen oder Viren nachgewiesen werden.

Bevorzugt ist auch ein solches Verfahren, wobei Antigene verschiedener Mikroorganismen oder Viren mittels einer Reaktion mit verschiedenen polyklonalen oder monoklonalen Antikörpern, die entsprechend ihrer Spezifität für Antigene verschiedener Mikroorganismen unterschiedliche Markierungen tragen, getrennt nachgewiesen werden können.

Ganz besonders bevorzugt ist dabei ein Verfahren, wobei die feste Phase eine Mikrotitrationsplatte ist.

Ganz besonders bevorzugt ist auch ein Verfahren, wobei die feste Phase eine Membran aus Nylon oder Nitrocellulose, bevorzugterweise aus Nitrocellulose, ist.

Durch die Verwendung polyklonaler Seren gegen möglichst alle Antigene/Epitope des Wirkstoffes im Impfstoff oder spezifischer polyklonaler oder monoklonaler Antikörper gegen bestimmte Antigene/Epitope lassen sich z.B. bestimmte Antigene eines Virus oder bestimmte Mikroorganismen in einer Mischung (Kombinationsimpfstoffe) oder der Anteil des Trägers einer Polysaccharid-Konjugat-Vaccine quantitativ differenzieren.

Zur Durchführung des erfindungsgemäßen Verfahrens werden z. B. Referenz-Antigen und Probe-Antigen in einem dem Fachmann bekannten wässrigen Lösungsmittel, wie z. B. gepufferter oder auch ungepufferter Lösung, die ein Neutralsalz enthält, bevorzugterweise gepufferter isotonischer NaCl Lösung, die auch Detergenz enthalten kann, gelöst und in Kontakt gebracht mit einer Trägermatrix, die Antigene physikalisch adsorbieren kann; bevorzugt sind dabei solche feste Phasen, die aus Polystyrol, Nylon oder Zellulosenitrat bestehen. Die Form der festen Phase kann in weiten Grenzen variieren, bevorzugt sind partikuläre Phasen, Membranen und Mikrotitrationsplatten. Vorteilhafterweise sind die partikulären Phasen magnetisierbar.
Bevorzugt ist die Verwendung von Membranen aus Nylon oder Nitrocellulose.

Physikalische Adsorption im Sinne der vorliegenden Erfindung bedeutet, daß die Bindung nicht durch immunchemische Reaktion erfolgt, sondern z. B. durch solche Kräfte, die dem Fachmann als von der Waal'sche Kräfte oder Wasserstoffbrückenbindungen bekannt sind.

Referenz-Antigene und Probe-Antigene werden in Verdünnungsstufen mit einem Faktor von 1.1 bis 20, vorzugsweise von 1,2 bis 8, insbesondere von 1,5 bis 3, aufgetragen, wobei die Antigen(Protein)konzentration des nachzuweisenden Antigens nicht höher als 5 µg, vorzugsweise 0,2 bis 0,01 µg/Auftragung zu Beginn der Verdünnungsreihe betragen sollte. Das Antigen reagiert mit einem spezifischen Antiserum, gereinigtem IgG oder IgM oder monoklonalen Antikörpern in Arbeitsverdünnungen von z. B. 1:50 bis 1:50.000 in Abhängigkeit von der Konzentration der Immunglobuline in der Serumpräparation. Diese Antikörper können durch dem Fachmann an sich bekannten Reaktionen direkt mit einer nachweisbaren Markierung konjugiert werden. In einem bevorzugten Verfahren werden diese spezifischen Antikörper wiederum mit einem mit einer nachweisbaren Markierung konjugierten zweiten Antikörper in dem Fachmann für solche Bestimmungen bekannten Arbeitsverdünnungen nachgewiesen.

Solche nachweisbaren Markierungen sind dem Fachmann an sich bekannt; dazu zählen z. B. Enzyme und fluoreszierende und chemolumineszierende Gruppen.

Bevorzugt ist, insbesondere in Verbindung mit der Verwendung von magnetisierbaren Partikeln, die Verwendung einer chemolumineszenten Markierung.

Das durch den Nachweis der Markierung erhaltene Signal wird mit dem Fachmann bekannten Methoden gemessen; durch Vergleich mit dem Referenzantigen wird der Antigen-Gehalt der Probe bestimmt. Unspezifische Reaktionen des 1. Antikörpers mit unerwünschten Begleitproteinen in der Probe können z. B. durch Inkubation dieses Antikörpers mit diesen Begleitproteinen minimiert werden; diese unerwünschte Seroreaktion kann auftreten, wenn z. B. der 1. Antikörper durch Antigen induziert wurde, das identisch oder sehr ähnlich hergestellt wurde wie das Antigen in der Probe - ein heterologes Produktionssystem des Antigens für den 1. Antikörper ist, wenn praktisch durchführbar, vorzuziehen.

Das in den Beispielen verwendete Begrivac ^{R} (Behringwerke AG, Marburg, Deutschland) enthält jeweils drei von der WHO empfohlene Influenzavirusstämme; der Impfstoff 1991 enthält die Stämme A/Singapore, A/Beijing und B/Yamagata. Der Antigengehalt wurde auf jeweils 34 µg Hämagglutinin der 3 Stämme/ml Impfdosis aufgrund des SRD-Tests am nicht inaktivierten Virusmaterial eingestellt. Fig. 4 zeigt die Ergebnisse des SRD-Tests und des erfindungsgemäßen Verfahrens für den Antigengehalt im (inaktivierten) Impfstoff.

Die folgenden Beispiele erläutern die Erfindung, schränken sie aber in keiner Weise ein.

### Beispiel 1

Bestimmung des Hämagglutinins (HA) in kommerziellen Vaccinen mittels Immuno-Dot-Blot:

### Material:

Begrivac^{R} 91 und Begrivac^{R} 92 (zu prüfende Vaccine) (Behringwerke AG)
Referenz-Antigene vom National Institute for Biological Control and Standarization, Potters Bar, UK
Referenz-Antiseren vom National Institute for Biological Control and Standarization, Potters Bar, UK
Blotting-Membranen (z.B. Cellulosenitrat (SCHLEICHER & SCHUELL, Dassel, Deutschland) BA 85/0,45 µm oder Nylon-Membran (Millipore, Eschborn, Deutschland) Immobilon-Membran (PVDF), IPUH 00010/0,45 µm)

Referenz-Antigene aller 3 Influenzastämme werden auf 34 µl HA/ml sowohl für jeden einzelnen Stamm getrennt als auch für die Mischung der Stämme als trivalente Kombination verdünnt.

Referenz-Antigene und Kombination sowie Influenza-Vaccinein werden in einer Mikrotiterplatte in geometrischen Verdünnungsreihen (Vorverdünnung 1:20; Faktor der Verdünnungsreihe 2; Verdünnungspuffer 25 mM Tris/192 mM Glycin) je 3 mal angelegt.

Die Blotting-Apparatur (z.B. Schleicher & Schuell) wird nach der Betriebsanleitung des Herstellers zusammengebaut.

In jedes Loch der Blotting-Apparatur werden 100 µl Verdünnungspuffer pipettiert. Nach Absaugen mittels Vacuum wird in jedes Loch der Blotting-Apparatur 100 µl der Proben-Verdünnungen aus der Mikrotiterplatte übertragen und mittels Vacuum abgesaugt; das Antigen bindet an die Membran.

Die Membran wird 2 h bei Raumtemperatur in 30 ml 3 %iger Magermilch-Lösung in PBS inkubiert (um die proteinbindene Kapazität der Membran zu blocken).

Referenz-Antiserum (1. Antikörper gegen HA) wird in 30 ml 0,5 %iger Ovalbumin-Lösung in PBS (PBS ohne Ca²⁺/Mg²⁺) mit 5 % Allantoisflüssigkeit aus gesunden Hühnereiern verdünnt - zur Bestimmung von A/Singapore: Arbeitsverdünnung 1:20.000; von A/Beijing: Arbeitsverdünnung 1:15.000; von B/Yamagata: Arbeitsverdünnung 1:32.000 - und die Membran in dieser Lösung über Nacht bei Raumtemperatur auf einem Wipptisch inkubiert.

### Waschen der Membran

- 30 min in PBS mit 0,1 % Triton X 100
- 5 min in PBS mit 1 M NaCl
- 30 min in PBS mit 0,1 % Triton X 100

Die Membran wird in 30 ml 0,5 %iger Magermilch-Lösung in PBS mit 2. Antikörper (Anti-Sheep-POD (Sigma Chemie, Deisenhofen, Deutschland; Best.-Nr. A 3415)) 1:2.500 verdünnt und 2 h bei Raumtemperatur inkubiert.

### Waschen der Membran wie oben

Die Membran wird in 30 ml Färbelösung (3 ml 4-Chlor-1-naphthol (3 mg/ml Äthanol)/15 ml 50 mM Tris/HCl, pH 7,6/12 ml PBS/15 µl H₂O₂) 10 min bei Raumtemperatur in der Dunkelheit inkubiert.

Die Membran wird nach der Färbung mehrmals in destilliertem Wasser gewaschen.

Zur quantitativen Bestimmung werden die Blots mit einem Laserscanner (Molecular Dynamics, Sunnyvale, CA, USA, Software: protein + dna imageware systems, pdi, Huntigton Station, NY, USA) gescannt. Die Mittelwerte der Farbintensität/Dot (Optische Dichte/Fläche) der Mehrfachbestimmungen werden graphisch für Referenz und Probe aufgetragen. Mit einer ELISA-Software wird durch Anlegen von Cut Offs im Arbeitsbereich im Vergleich zur Referenz der HA-Gehalt der Proben bestimmt. (Fig. 1 und 2)

### Beispiel 2

Bestimmung des Hämagglutinins (HA) in kommerziellen Vaccinen mittels ELISA:

### Material:

Referenz- und Proben-Material wie bei Beispiel 1
Mikrotiter-Platten für ELISA (z.B. Greiner, Frickenhausen, Deutschland)

Referenz-Antigene aller 3 Influenza-Stämme werden auf 34 µl HA/ml sowohl für jeden einzelnen Stamm getrennt als auch für die Mischung der Stämme als trivalente Kombination verdünnt.

Je 100 µl Referenz-Antigene und Kombination sowie Influenza-Vaccinen werden in einer ELISA-Mikrotiterplatte in geometrischen Verdünnungsreihen (Vorverdünnung 1:100; Faktor der Verdünnung 2; Verdünnungspuffer 0,05 M Na₂CO₃, pH 9,6) je 2 mal angelegt. Die Platte wird über Nacht bei Raumtemperatur inkubiert.

Die Platte wird 3 mal mit Waschpuffer (0,5 % Magermilchpulver und 0,05 % Tween 20 in PBS pH 7,2) gewaschen und in jeden Napf der Mikrotiterplatte 100 µl des 1. Antikörpers (Anti-Singapore), 1:5000 in Waschpuffer mit 5 % Allantoisflüssigkeit aus gesunden Hühnereiern verdünnt, gegeben.

Nach 1 h Inkubation bei Raumtemperatur wird die Platte 3 mal mit Waschpuffer gewaschen und der 2. Antikörper (Anti-Schaf-POD markiert; Sigma Chemie) zugegeben (100 µl/Napf, 1:2500 in Waschpuffer verdünnt).

Die Platte wird 4 mal mit Waschpuffer gewaschen und danach 100 µl Substratlösung/Napf einpipettiert (Substratlösung: 150 mg o-Phenylendiamin in 100 ml Phosphat-Citrat-Puffer und 50 µl H₂O₂).

Nach Inkubation von 30 min bei Raumtemperatur im Dunkeln wird die Reaktion durch Zugabe von 100 µl 1 M H₂SO₄/Napf gestoppt. Die Farbintensität wird in einem geeigneten ELISA-Reader gemessen und ggf. der HA-Gehalt anhand der Referenz bestimmt (Fig. 3).

## Patentansprüche

1. Verfahren zur immunchemischen Quantifizierung von inaktivierten immunreaktiven Hämagglutinin-Molekülkomplexen von Influenzaviren, das folgende Schritte umfaßt:
a) eine Probe, die einen oder mehrere zu bestimmende Hämagglutinin-Molekülkomplexe enthält, die während der Spaltung der Viren durch Lipidlösungsmittel und nachfolgender Inaktivierung mit Formaldehyd entstanden sind, wird mit einer proteinbindenden festen Phase inkubiert, wobei Hämagglutinin- Molekülkomplex an diese feste Phase physikalisch adsorbiert und nicht durch immunchemische Reaktion gebunden wird,
b) die flüssige und die feste Phase werden getrennt,
c) der adsorbierte Hämagglutinin-Molekülkomplex wird mit einem oder mehreren spezifischen Antikörpern, die direkt oder indirekt eine Markierung tragen, inkubiert,
d) die Menge der an den Hämagglutinin-Molekülkomplex gebundenen Markierung wird bestimmt, und
e) aus der Menge der gebundenen Markierung wird durch Vergleich mit einem oder mehreren Standardwerten die Menge des immunreaktiven Hämagglutinin-Molekülkomplexes bestimmt.

2. Verfahren nach Anspruch 1, wobei die Probe Hämagglutinin-Molekülkomplexe von drei verschiedenen Influenzaviren enthält.

3. Verfahren nach Anspruch 1, wobei die Hämagglutinin-Molekülkomplexe eines Influenzavirus mittels Reaktion mit einem polyklonalen Antikörper oder einem monoklonalen Antikörper oder einer Mischung von monoklonalen Antikörpern, die die gleiche Markierung tragen, nachgewiesen werden.

4. Verfahren nach Anspruch 1, wobei die Hämagglutinin-Molekülkomplexe eines Influenzavirus mittels einer Reaktion mit spezifischen polyklonalen oder monoklonalen Antikörpern aus einer Mischung verschiedener Mikroorganismen oder Viren nachgewiesen werden.

5. Verfahren nach Anspruch 1, wobei Hämagglutinin- Molekülkomplexe verschiedener Influenzaviren mittels einer Reaktion mit verschiedenen polyklonalen oder monoklonalen Antikörpern, die entsprechend ihrer Spezifität für Hämagglutinin-Molekülkomplexe verschiedener Influenzaviren unterschiedliche Markierungen tragen, getrennt nachgewiesen werden.

6. Verfahren nach Anspruch 1, wobei die feste Phase aus Polystyrol, Nylon oder Nitrocellulose besteht.

7. Verfahren nach Anspruch 1, wobei die feste Phase partikulär ist.

8. Verfahren nach Anspruch 1, wobei die feste Phase eine Mikrotitrationsplatte ist.

9. Verfahren nach Anspruch 1, wobei die feste Phase eine Membran aus Nylon oder Nitrocellulose, bevorzugterweise aus Nitrocellulose, ist.

## Claims

1. Method for the immunochemical quantification of inactivated immunoreactive haemagglutinin molecule complexes of influenza viruses, which method comprises the following steps:
a) a sample which contains one or more haemagglutinin molecule complexes to be determined, which complexes have been formed during cleavage of the viruses by lipid-solubilizing agents and subsequent inactivation with formaldehyde, is incubated with a protein-binding solid phase, with haemagglutinin molecule complex being adsorbed physically to this solid phase and not being bound by immunochemical reaction,
b) the liquid and the solid phases are separated,
c) the adsorbed haemagglutinin molecule complex is incubated with one or more specific antibodies which, directly or indirectly, carry a label,
d) the quantity of the label bound to the haemagglutinin molecule complex is determined, and
e) the quantity of the immunoreactive haemagglutinin molecule complex is determined from the quantity of the bound label by comparison with one or more standard values.

2. Method according to Claim 1, wherein the sample contains haemagglutinin molecule complexes of three different influenza viruses.

3. Method according to Claim 1, wherein the haemagglutinin molecule complexes of an influenza virus are detected by means of reaction with a polyclonal antibody or a monoclonal antibody, or a mixture of monoclonal antibodies which carry the same label.

4. Method according to Claim 1, wherein the haemagglutinin molecule complexes of an influenza virus are detected by means of a reaction with specific polyclonal or monoclonal antibodies from a mixture of different microorganisms or viruses.

5. Method according to Claim 1, wherein haemagglutinin molecule complexes of different influenza viruses are detected separately by means of a reaction with different polyclonal or monoclonal antibodies which, corresponding to their specificity for haemagglutinin molecule complexes of different influenza viruses, carry different labels.

6. Method according to Claim 1, wherein the solid phase is composed of polystyrene, nylon or nitrocellulose.

7. Method according to Claim 1, wherein the solid phase is particulate.

8. Method according to Claim 1, wherein the solid phase is a microtitration plate.

9. Method according to Claim 1, wherein the solid phase is a nylon membrane or nitrocellulose membrane, preferably a nitrocellulose membrane.

## Revendications

1. Procédé pour la quantification immunochimique de complexes moléculaires d'hémagglutinine immunoréactifs inactivés de l'influenzavirus, comprenant les étapes suivantes :
a) un échantillon qui contient un ou plusieurs complexes moléculaires d'hémagglutinine à déterminer, qui est/sont apparu(s) pendant la dissociation des virus par un solvant des lipides et l'inactivation consécutive par le formaldéhyde, est incubé avec une phase solide liant les protéines, le complexe moléculaire d'hémagglutinine s'adsorbant physiquement sur cette phase solide et n'étant pas lié par une réaction immunochimique,
b) les phases solide et liquide sont séparées,
c) le complexe moléculaire d'hémagglutinine adsorbé est incubé avec un ou plusieurs anticorps spécifiques qui portent directement ou indirectement un marquage,
d) la quantité de marquage lié au complexe moléculaire d'hémagglutinine est déterminée, et
e) à partir de la quantité de marquage lié, la quantité de complexe moléculaire d'hémagglutinine immunoréactif est déterminée par comparaison avec une ou plusieurs valeurs standard.

2. Procédé selon la revendication 1, dans lequel l'échantillon contient des complexes moléculaires d'hémagglutinine de trois influenzavirus différents.

3. Procédé selon la revendication 1, dans lequel les complexes moléculaires d'hémagglutinine d'un influenzavirus sont détectés par l'intermédiaire d'une réaction avec un anticorps polyclonal ou avec un anticorps monoclonal ou avec un mélange d'anticorps monoclonaux qui portent le même marquage.

4. Procédé selon la revendication 1, dans lequel les complexes moléculaires d'hémagglutinine d'un influenzavirus sont détectés par l'intermédiaire d'une réaction avec des anticorps monoclonaux ou polyclonaux spécifiques issus d'un mélange de différents micro-organismes ou virus.

5. Procédé selon la revendication 1, dans lequel les complexes moléculaires d'hémagglutinine de différents influenzavirus sont détectés de manière distincte par l'intermédiaire d'une réaction avec des anticorps monoclonaux ou polyclonaux différents qui portent différents marquages en fonction de leur spécificité pour des complexes moléculaires d'hémagglutinine de différents influenzavirus.

6. Procédé selon la revendication 1, dans lequel la phase solide se compose de polystyrène, de Nylon ou de nitrocellulose.

7. Procédé selon la revendication 1, dans lequel la phase solide est particulaire.

8. Procédé selon la revendication 1, dans lequel la phase solide est une plaque de microtitrage.

9. Procédé selon la revendication 1, dans lequel la phase solide est une membrane en Nylon ou en nitrocellulose, de préférence en nitrocellulose.
